# FASCICULE DE BREVET EUROPEEN

(11) **EP 1 005 570 B1**
(45) Date de publication et mention de la délivrance du brevet: **25.10.2006**
(21) Numéro de dépôt: 98941517.9
(22) Date de dépôt: 31.07.1998
(51) Int. Cl.: C12Q 1/04, C12Q 1/34

(54) **MILIEUX DE CULTURE ET D'IDENTIFICATION SPECIFIQUE DE DIFFERENTES ESPECES DE CANDIDA ET PROCEDES D'ANALYSE.**
ZELLKULTURMEDIEN ZUM SPEZIFISCHEN NACHWEIS VON VERSCHIEDENEN CANDIDA-SPEZIES, SOWIE TESTMETHODEN
CULTURE AND IDENTIFICATION MEDIA SPECIFIC FOR DIFFERENT SPECIES OF CANDIDA AND ANALYSIS METHODS.

(30) Priorité: 20.08.1997 FR 9710635; 20.04.1998 FR 9805269
(43) Date de publication de la demande: 07.06.2000
(60) Demande divisionnaire: 05002486.8
(73) Titulaire: BIO MERIEUX, 69280 Marcy l'Etoile (FR)
(72) Inventeur: ORENGA, Sylvain, F-01160 Neuville sur Ain (FR)
(74) Mandataire: Guerre, Dominique
(86) Numéro de dépôt international: PCT/FR1998/001717
(87) Numéro de publication internationale: WO 1999/009207

(56) Documents cités:
- EP-A- 0 544 605
- FR-A- 2 659 982
- US-A- 5 120 718
- BENDEL C M ET AL: "Distinct mechanism of epithelial adhesion for Candida albicans and Candida tropicalis: Identification of the participating ligands and development of inhibitory peptides." JOURNAL OF CLINICAL INVESTIGATION 92 (4). 1993. 1840-1849. ISSN: 0021-9738, XP002064132
- MIRA-GUTIEREZ J ET AL: "Identification of yeast by hydrolysis of amides." MYCOSES 38 (3-4). 1995. 101-106. ISSN: 0933-7407, XP002064133
- CRIST A E JR ET AL: "Comparison of the MUREX C. albicans, albicans-sure, and BactiCard Candida test kits with the germ tube test for presumptive identification of Candida albicans." JOURNAL OF CLINICAL MICROBIOLOGY 34 (10). 1996. 2616-2618. ISSN: 0095-1137, XP002064134
- HEELAN J S ET AL: "Comparison of rapid testing methods for enzyme production with the germ tube method for presumptive identification of Candida albicans." JOURNAL OF CLINICAL MICROBIOLOGY 34 (11). 1996. 2847-2849. ISSN: 0095-1137, XP002064135
- GARCIA-MARTOS P ET AL: "Contribution to the knowledge of the enzymatic activity of yeasts of clinical interest." MYCOPATHOLOGIA 132 (1). 1995. 9-13. ISSN: 0301-486X, XP002064136
- NICOLAUS B.J.R.: 'Symbiotic Approach to Drug Design' DECISION MAKING IN DRUG RESEARCH 1983, pages 173 - 186

## Description

La présente invention concerne un milieu de culture et d'identification spécifique de levures et un procédé d'analyse microbiologique pour identifier spécifiquement les levures *Candida albicans* et *Candida tropicalis* et/ou différencier les levures *C. albicans* et *C. tropicalis.*

L'espèce *C. albicans* est la plus communément isolée à partir d'échantillons cliniques et provoque des infections plus ou moins importantes de la peau, des ongles et des muqueuses chez les individus présentant des défenses immunitaires normales et des infections très sérieuses chez les individus affaiblis et notamment ceux infectés par le Virus de l'Immunodéficience Humaine (VIH). Selon les études *C. tropicalis* est la deuxième ou troisième espèce en fréquence d'isolement dans les prélèvements d'origine humaine. Il est donc essentiel non seulement de pouvoir détecter très rapidement la présence de ces levures dans des prélèvements, mais également de différencier celles appartenant à l'espèce *C. albicans,* de celles de l'espèce *C. tropicalis.*

Pour cela, il a été proposé ces dernières années de nombreuses techniques pour identifier rapidement les levures *C. albicans.* Le plus grand nombre d'entre elles est basé sur la mise en évidence d'une activité hexosaminidase, c'est-à-dire d'enzymes ayant une activité N-acétyl-β-D-glucosaminidase ou N-acétyl-β-D-galactosaminidase ou N-acétyl-β-D-mannosaminidase (FR-2 684 110, FR-2 659 982). Néanmoins ces procédés souffrent d'une spécificité réduite vis-à-vis des levures de l'espèce *C. tropicalis.*

Les inventeurs de la présente invention ont découvert qu'en inhibant une activité enzymatique de l'espèce *C. tropicalis,* notamment l'activité hexosaminidase, il était possible de pallier aux inconvénients des tests précités et ainsi d'apporter un moyen d'identification et/ou de différenciation des levures, notamment de *C. albicans* et *C*. *tropicalis,* rapide et peu coûteux.

Par ailleurs, l'activité enzymatique glucosidase à déjà fait l'objet de recherches par certains documents, comme Casal, M. et Linares, M.J. « Contribution to the study of the enzymatic profiles of yeast organisms with medical interest » Mycopathologie 81,155-159 (1983). Cette activité est positive chez quelques souches de *C. albicans, C. tropicalis* et *Candida pseudotropicalis* (appelé aujourd'hui Candida kefyr), mais négatives pour les autres *Candida, par exemple C. parapsilosis, C. guilliermondii, C. krusei.*

Il est donc apparu intéressant d'essayer de cumuler, dans un même milieu, la possibilité de rechercher deux activités enzymatiques différentes, c'est-à-dire hexosaminidase et glucosidase. Or on constate que, dans les milieux selon l'invention, ce cumul permet de différencier plus précisément les *C. albicans* par rapport aux *C. guilliermondii, C. kefyr, C. lusitaniae* et/ou *C. tropicalis* et par rapport aux autres *Candida,* mais également de différencier les *C. guilliermondii, C. kefyr, C. lusitaniae* et/ou *C. tropicalis* par rapport aux autres *Candida*.

Bien entendu, il est prévu d'associer, dans un même milieu, aux substrats spécifiques des activités hexosaminidase et glucosidase, que l'on recherche, un inhibiteur selon l'invention, et même un activateur de l'activité hexosaminidase.

L'objet de l'invention est donc un milieu de culture et d'identification spécifique de levures comprenant un substrat chromogène ou fluorigène, susceptible d'être hydrolysé par une enzyme du groupe des hexosaminidases, caractérisé en ce que le milieu comprend en outre au moins un composé sélectivement inhibiteur de l'activité hexosaminidase de *Candida tropicalis.*

Grâce à l'invention, le milieu de culture permet notamment l'identification spécifique des levures de l'espèce *C. albicans* et/ou *C. tropicalis.*

Selon un mode de réalisation préférentiel de l'invention, le milieu de culture comprend en tant que composé sélectivement inhibiteur une amide de formule (I):

(I) R-(CO-NR'R'')ₙ

dans laquelle, premièrement, soit R, R' et R'' sont, indépendamment les uns des autres, constitués par :
- un atome d'hydrogène,
- une chaîne hydrocarbonée, saturée ou insaturée, aliphatique ou cyclique, comportant éventuellement au moins un hétéroatome,
soit chacun des R et/ou R' et/ou R" forment ensemble une chaîne hydrocarbonée cyclique, saturée ou insaturée, comportant éventuellement au moins un hétéroatome,
et deuxièmement, n est un nombre entier supérieur ou égal à 1.

Selon l'invention, par une chaîne hydrocarbonée "comportant" au moins un hétéroatome, on entend que la chaîne hydrocarbonée peut être substituée par au moins un substituant tel que notamment -NH₂, -COOH, -SH, et un atome d'halogène, et/ou peut être interrompue par au moins un hétéroatome tel que notamment O, S, et N.

Selon un mode de réalisation préférentiel de l'invention, le milieu de culture comprend en tant que composé sélectivement inhibiteur une amide de formule (I):

(I) R-(CO-NR'R'')ₙ

dans laquelle, premièrement, soit R, R' et R" sont, indépendamment les uns des autres, constitués par :
- un atome d'hydrogène,
- une chaîne hydrocarbonée, saturée ou insaturée, aliphatique ou cyclique, éventuellement interrompue par au moins un hétéroatome,
soit chacun des R et/ou R' et/ou R'' forment ensemble une chaîne hydrocarbonée cyclique, saturée ou insaturée, comportant éventuellement au moins un hétéroatome,
et deuxièmement, n est un nombre entier supérieur ou égal à 1.

Selon un autre mode de réalisation préférentiel de l'invention, le milieu de culture comprend en tant que composé sélectivement inhibiteur une amide de formule (I):

(I) R-(CO-NR'R")ₙ

dans laquelle, premièrement, R, R' et R" sont, indépendamment les uns des autres, constitués par :
- un atome d'hydrogène,
- une chaîne hydrocarbonée aliphatique,
et, deuxièmement, n est égal à 1 ou 2.

Selon un mode de réalisation très préférentiel de l'invention, le composé sélectivement inhibiteur est une acétamide.

Selon un autre mode de réalisation de l'invention, le milieu de culture comprend un activateur spécifique de l'enzyme hexosaminidase de *C. albicans.*

Selon un mode de réalisation préféré de l'invention, l'activateur spécifique de l'enzyme hexosaminidase est la N-acétyl-glucosamine.

Selon un autre mode de réalisation de l'invention, le milieu de culture comprend un mélange de composés sélectivement inhibiteurs.

Selon un mode de réalisation préféré de l'invention, le mélange de composés sélectivement inhibiteurs est constitué d'acétamide et de formamide.

Selon un mode de réalisation préféré de l'invention, le milieu est liquide ou gélifié.

Selon un mode de réalisation de l'invention, le milieu de culture est gélifié et comprend pour 1 litre :
- peptones ou mélange de peptones 0,01-40 g
- extrait de levure 0,01-40 g
- glucose (source de carbone) 0-10 g
- tampon phosphate (pH entre 5 et 8,5) 2,5-100 mM
- 5-Bromo-4-chloro-3-indolyl-N-acétyl-β-D-glucosaminide 20-600.10⁻⁶ M
- acétamide 0,01-20 g
- inhibiteur de bactéries 0-20 g
- agar 11-20 g

Selon un autre mode de réalisation préféré de l'invention, le milieu de culture gélifié ou liquide décrit ci-dessus comprend de plus de la N-acétyl-glucosamine à 1,0 g/l.

Selon un autre mode de réalisation préféré de l'invention, le milieu de culture gélifié ou liquide décrit ci-desssus comprend de plus de la formamide à 0,5 g/l.

Un autre objet de l'invention est un procédé d'analyse microbiologique pour identifier sélectivement les levures *C. albicans* et/ou *C. tropicalis* et/ou différencier les levures *C. albicans* et *C. tropicalis,* caractérisé en ce que l'on met directement l'échantillon à analyser au contact d'au moins un milieu d'identification décrit ci-dessus.

A cet effet la présente invention concerne également un milieu pour la détection et l'identification spécifique de levures, qui est caractérisé par le fait qu'il comprend deux substrats, un premier substrat, chromogène ou fluorigène, susceptible d'être hydrolysé par une enzyme du groupe des hexosaminidases, et un second substrat, chromogène ou fluorigène, susceptible d'être hydrolysé par une enzyme du groupe des glucosidases.

Selon un mode préféré de réalisation de l'invention, dans ce milieu, chaque substrat est constitué d'une partie spécifique de l'enzyme et d'une partie marqueur, caractérisé par le fait que la partie marqueur du premier substrat est différente de la partie marqueur du second substrat.

Selon un autre mode préféré de réalisation de l'invention, le milieu comprend un activateur et/ou un inhibiteur d'hexosaminidase.

Dans le cas où il y a un activateur et/ou un inhibiteur, cet activiteur est constitué par une hexosamine et/ou une hexosaminidine et cet inhibiteur reprend les caractéristiques décrites ci-dessus.

Selon encore un autre mode préféré de réalisation de l'invention, le substrat d'hexosaminidinase est constitué par un dérivé d'indoxyl et/ou le substrat de glucosidase est constitué par un dérivé d'indoxyl.

Dans tous les cas de figure, le milieu est liquide ou gélifié.

La présente invention concerne encore un procédé d'analyse microbiologique pour détecter et identifier sélectivement certaines espèces de levures *Candida,* qui est caractérisé en ce que l'on met directement l'échantillon en contact avec un milieu selon l'une quelconque des revendications 13 ou 18, que l'on attend que des colorations apparaissent dans le milieu et que l'on identifie, par des différences de colorations, les *C. albicans* par rapport, d'une part, aux *C. guilliermondii, C. kefyr, C. lusitaniae* et/ou *C. tropicalis* et, d'autre part, aux autres *Candida,* ainsi que les *C. guilliermondii, C. kefyr, C. lusitaniae* et/ou *C. tropicalis* par rapport aux autres *Candida*.

L'on attend entre 36 et 60 heures et avantageusement sensiblement 48 heures, lorsque le milieu ne contient pas d'activateur ou d'inhibiteur.

L'on attend entre 18 et 30 heures et avantageusement sensiblement 24 heures, lorsque le milieu contient un activateur ou un inhibiteur.

Selon un premier mode de réalisation, ces procédés permettent d'identifier *C. albicans, C. guilliermondii, C. kefyr, C. lusitaniae* et/ou *C. tropicalis* par rapport aux autres *Candida,* lorsque le milieu contient :
- un substrat d'hexosaminidase, et/ou
- un substrat de glucosidase, et/ou
- un activateur d'hexosaminidase, et/ou
- inhibiteur d'hexosaminidase.

Selon un deuxième mode de réalisation, ces procédés permettent d'identifier *C. albicans* par rapport aux *C. guilliermondii, C. kefyr, C. lusitaniae, C. tropicalis* et/ou aux autres *Candida*, lorsque le milieu contient :
- un substrat d'hexosaminidase et un substrat de glucosidase, et/ou
- un activateur d'hexosaminidase, et/ou
- inhibiteur d'hexosaminidase.

Par "composé sélectivement inhibiteur de l'activité de l'hexosaminidase de *C. tropicalis",* on entend tout composé capable d'inhiber de manière sélective l'activité de l'hexosaminidase de *C. tropicalis.* Par exemple, les composés de type amide de formule décrite ci-dessus ont la propriété d'inhiber spécifiquement l'activité hexosaminidase des *C. tropicalis,* sans affecter celle des *C. albicans.*

Par "identification", on entend la détection et/ou la quantification.

Par "échantillon", on entend notamment tout prélèvement de type biologique, une souche ou un ensemble de souches de levure isolées par exemple après culture.

On expose ci-après, de manière générale la composition du milieu de culture, exprimée en g/l de milieu final.

Le milieu comprend une base nutritive nécessaire au développement des levures et des inhibiteurs spécifiques de l'hexosaminidase des *C. tropicalis* selon l'invention.

Les éléments constitutifs de la base nutritive comprennent :
- des peptones de 0,01 à 40 g/l, telles que la peptone de viande, le produit commercialisé par la société bioMérieux sous la marque bioSoyase ou analogue, ou encore un mélange de peptones ; de préférence, la peptone ou le mélange de peptones est présent dans le milieu à environ 6 g/l ± 0,5 g/l ;
- un extrait de levure de 0,01 à 40 g/l, de préférence environ 1,5 g/l, apportant des vitamines de croissance des levures ;
- une source de carbone, telle que le glucose, le glycérol, un acétate, un pyruvate, un lactate, l'arginine, un aminobutyrate, ou un mélange de ces composants, dans la proportion de 0 à 10 g/l ; la source de carbone est de préférence le glucose en une quantité de 1 g/l ;
- un tampon ajouté au milieu afin d'obtenir un pH favorable pour le développement de *C. albicans,* compris entre 5 et 8,5 ; le tampon est choisi parmi les tampons phosphates, Tris, Hépès (acide N-2-hydroxyéthyl-pipérazine-N'-2-éthasulfonique) et citrate dans la proportion de 2,5 à 100 mM ; de préférence, le tampon est un tampon phosphate 10 mM pour ajuster le pH du milieu à une valeur voisine de 7 ;
- de l'Agar de 11 à 20 g/l, de préférence de 15 g/l.

Le substrat chromogène ou fluorigène peut être tout substrat chromogène ou fluorigène hydrolysable par une hexosaminidase, telle qu'une galactosaminidase, glucosaminidase ou mannosaminidase, pour libérer un produit coloré ou fluorescent. De préférence, le substrat est choisi parmi ceux présentant une forte coloration ou fluorescence avec peu de molécules, et n'induisant pas de modification du métabolisme des microorganismes, excepté pour l'activité enzymatique recherchée. Ces substrats sont de préférence choisis, pour les substrats chromogènes, parmi ceux comprenant un groupement chromophore tel qu'un indolyle substitué ou non, et notamment parmi le 5-Bromo-4-chloro-3-indolyl-N-acétyl-β-D-glucosaminide, le 5-Bromo-4-chloro-3-indolyl-N-acétyl-β-D-galactosaminide, 6-Chloro-3-indolyl-N-acétyl-N-D-glucosaminide ou le 5-Bromo-6-chloro-3-indolyl-N-acétyl-β-D-glucosaminide de 20 à 600 mM, avantageusement le 5-Bromo-4-chloro-3-indolyl-N-acétyl-β-D-glucosaminide à 200 mM, et pour les substrats fluorigènes, parmi la 4-Méthylumbelliféryl-N-acétyl-b-D-galactosaminide, la 4-Méthylumbelliféryl-N-acétyl-b-D-glucosaminide.

L'inhibiteur spécifique de l'hexosaminidase des levures de l'espèce *C. tropicalis* est choisi préférentiellement dans le groupe des composés de type amide (I) ou leurs mélanges. Il est notamment choisi parmi les amides telles que la formamide, l'acétamide, la propionamide, la glycinamide, la succinamide, et autres. La quantité du composé de type amide est comprise entre 0,01 et 20 g/l. De préférence l'inhibiteur choisi est l'acétamide à 1 g/l.

Afin d'obtenir pour les levures de l'espèce *C. albicans* une activité intense et précoce, il peut avantageusement être ajouté au milieu de culture un activateur d'hexosaminidase tel que décrit dans le document FR-A-2.684.110. De même un inhibiteur ou un mélange d'inhibiteurs des bactéries, permettant d'inhiber la croissance des bactéries à Gram positif et de celles à Gram négatif, sans affecter celle des levures, et si possible des champignons, peut être ajouté au milieu. De préférence, les inhibiteurs de bactéries sont choisis dans le groupe des antibiotiques tels que gentamicine, chloramphénicol, pénicilline, streptomycine, cycloheximide, néomycine, tétracycline, oxytétracycline ou un mélange d'antibiotiques, et/ou parmi la tellurite, un molybdate et analogues, ou leurs mélanges. Avantageusement, on choisit le chloramphénicol (0,5 g/l), ou un mélange de gentamicine (0,1 g/l) et de chloramphénicol (0,05 g/l). Il est également possible d'inhiber la croissance des bactéries en diminuant le pH du milieu jusqu'à un pH acide.

Comme cela est démontré par les exemples ci-après, la réaction d'hydrolyse enzymatique reste spécifique au delà de 24 heures d'incubation.

### Exemple 1 :

Des essais ont été réalisés pour tester l'effet de l'acétamide sur l'activité hexosaminidase des levures.

Deux milieux ont été préparés selon les techniques habituelles. Le premier milieu ci-après désigné Milieu I contient tous les éléments de la base nutritive, ainsi qu'un substrat chromogène d'une hexosaminidase et un mélange inhibiteur de bactéries.

La composition du Milieu I pour un litre de milieu final est la suivante :
- bioSoyase (bioMérieux) 6,0 g
- extrait de levure (bioMérieux) 1,5 g
- glucose (Merck) 1,0 g
- tampon phosphate (Merck) 10,0 mM
- Mn2+ (Merck) 1,0 mM
- 5-Bromo-4-chloro-3-indolyl-N-acétyl-ß-D-glucosaminide (Biosynth) 0,1 g
- gentamicine 0,1 g
- chloramphénicol 0,05 g
- agar (bioMérieux) 15,0 g

Le pH du milieu a été ajusté aux environs de 7.

Le second milieu appelé Milieu II correspond au milieu selon l'invention et contient tous les éléments ci-dessus décrits pour le Milieu I, plus l'inhibiteur spécifique de l'hexosaminidase des *C. tropicalis,* c'est-à-dire un composé acétamide (Sigma) à 1,0 g.

Sur ces deux milieux, 12 souches de levures ont été directement cultivées en boîte de Pétri. Les souches provenant de la collection de la demanderesse, appartiennent aux espèces suivantes : *C*. *albicans* (3 souches), *C*. *glabrata* (2 souches), *C. krusei* (1 souche), *C. parapsilosis* (1 souche), *C. tropicalis* (3 souches), *Saccharomyces cerevisiae* (1 souche), *Trichosporon spp.* (1 souche). Les boîtes ont été incubées à 37°C pendant 48 heures. Les colonies formées ont été examinées visuellement, respectivement après 24 et 48 heures d'incubation selon les interprétations suivantes :
- les colonies bleues correspondent à des souches produisant la N-acétyl-β-D-glucosaminidase appartenant *a priori* à l'espèce *C. albicans ;*
- les colonies blanches correspondent aux souches ne produisant pas l'enzyme précitée ou dont cette enzyme est inhibée par le composé de type amide, elles appartiennent donc à d'autres espèces de levures qui seront alors à identifier à l'aide des techniques habituelles.

Les résultats sont présentés dans le tableau I ci-après :

**TABLEAU I**

| | | Coloration | | | | | |
|---|---|---|---|---|---|---|---|
| | | à 24 heures | | | à 48 heures | | |
| Espèces | Milieu | Forte | Faible | Nulle | Forte | Faible | Nulle |
| *C. albicans* | I | 1* | 2 | - | 3 | - | - |
| | II | 1 | 2 | - | 3 | - | - |
| *C. glabrata* | I | - | - | 2 | - | - | 2 |
| | II | - | - | 2 | - | - | 2 |
| *C. krusei* | I | - | - | 1 | - | - | 1 |
| | II | - | - | 1 | - | - | 1 |
| *C. parapsilosis* | I | - | - | 1 | - | - | 1 |
| | II | - | - | 1 | - | - | 1 |
| *C. tropicalis* | I | - | - | 3 | 3 | - | - |
| | II | - | - | 3 | - | 1 | 2 |
| *S. cerevisiae* | I | - | - | 1 | - | - | 1 |
| | II | - | - | 1 | - | - | 1 |
| *Trichosporon* | I | - | - | 1 | 1 | - | - |
| | II | - | - | 1 | 1 | - | - |

| | | | | | | | |
|---|---|---|---|---|---|---|---|
| * : nombre de souches, "-" = 0 | | | | | | | |

Comme cela ressort du tableau I ci-dessus, l'apport du composé de type amide permet une détection spécifique des souches de *C. albicans.* En effet, seules les souches de *C. albicans,* ainsi qu'une souche de *Trichosporon* après 48 heures d'incubation uniquement, produisent des colonies colorées sur le milieu selon l'invention. Les colonies de *C. tropicalis* qui sont bleues après 48 heures sur le Milieu I donnent des colonies incolores sur le Milieu II, sauf une très faiblement colorée après 48 heures d'incubation.

### Exemple 2 :

L'expérience de l'exemple 1 a été reproduite mais en utilisant des milieux liquides au lieu de milieux gélifiés. Les milieux III et IV correspondent donc aux milieux I et II de l'exemple 1 mais sont dépourvus d'agar. Par ailleurs, la concentration du 5-Bromo-4-chloro-3-indolyl-N-acétyl-b-D-glucosaminide est de 150 mg/l de milieu final pour une utilisation en milieu liquide. Les milieux ont été répartis en ampoules en verre, à raison de 3 ml par ampoule. Les souches étudiées sont les mêmes que dans l'exemple 1. Une suspension étalonnée à 2 MacFarland à l'aide d'un néphélomètre a été effectuée pour chacune des souches directement dans les ampoules contenant les milieux. Les ampoules ainsi inoculées ont été incubées 48 heures à 37°C. Elles ont été examinées après respectivement 24 et 48 heures selon les interprétations de l'exemple 1.

Les résultats sont présentés dans le tableau II ci-après :

**TABLEAU II**

| | | Coloration | | | | | |
|---|---|---|---|---|---|---|---|
| | | à 24 heures | | | à 48 heures | | |
| Espèces | Milieu | Forte | Faible | Nulle | Forte | Faible | Nulle |
| *C. albicans* | III | 1* | 2 | - | 3 | - | - |
| | IV | 1 | 2 | - | 3 | - | - |
| *C. glabrata* | III | - | - | 2 | - | - | 2 |
| | IV | - | - | 2 | - | - | 2 |
| *C. krusei* | III | - | - | 1 | - | - | 1 |
| | IV | - | - | 1 | - | - | 1 |
| C. parapsilosis | III | - | - | 1 | - | - | 1 |
| | IV | - | - | 1 | - | - | 1 |
| *C. tropicalis* | III | - | 2 | 1 | 3 | - | - |
| | IV | - | - | 3 | - | 2 | 1 |
| S. cerevisiae | III | - | - | 1 | - | - | 1 |
| | IV | - | - | 1 | - | - | 1 |
| *Trichosporon* | III | - | - | 1 | - | 1 | - |
| | IV | - | - | 1 | - | 1 | - |

| | | | | | | | |
|---|---|---|---|---|---|---|---|
| * : nombre de souches, "-" = 0 | | | | | | | |

Comme cela ressort du tableau II ci-dessus, l'apport du composé amidé permet une détection spécifique des souches de *C. albicans.* En effet après 24 heures d'incubation, seules les souches de *C. albicans* donnent des tubes colorés en bleu dans le milieu selon l'invention. Les souches de *C. tropicalis* qui donnent des tubes colorés dans le Milieu III donnent des tubes incolores dans le Milieu IV. Après 48 heures d'incubation la coloration des tubes comportant des souches de *C. tropicalis* est également inhibée ou au moins très fortement réduite.

### Exemple 3 :

Des essais ont été réalisés pour tester l'effet de l'acétamide sur l'activité hexosaminidase des levures en présence d'un activateur spécifique de cette enzyme.

L'expérience de l'exemple 1 a été reproduite mais en ajoutant au milieu de la N-Acétyl-glucosamine. Les milieux V et VI correspondent donc aux milieux I et II de l'exemple 1 auxquels on a ajouté de la N-Acétyl-glucosamine à 1,0 g/l de milieu final. Les souches étudiées sont les mêmes que dans l'exemple 1. Elles ont été directement cultivées en boîte de Pétri. Les boîtes ont été incubées à 37°C pendant 48 heures. Les colonies formées ont été examinées visuellement, respectivement après 24 et 48 heures d'incubation selon les interprétations de l'exemple 1.

Les résultats sont présentés dans le tableau III ci-après :

**TABLEAU III**

| | | Coloration | | | | | |
|---|---|---|---|---|---|---|---|
| | | à 24 heures | | | à 48 heures | | |
| Espèces | Milieu | Forte | Faible | Nulle | Forte | Faible | Nulle |
| *C. albicans* | V | 2* | 1 | - | 3 | - | - |
| | VI | 2 | 1 | - | 3 | - | - |
| *C. glabrata* | V | - | - | 2 | - | - | 2 |
| | VI | - | - | 2 | - | - | 2 |
| *C. krusei* | V | - | - | 1 | - | - | 1 |
| | VI | - | - | 1 | - | - | 1 |
| *C. parapsilosis* | V | - | - | 1 | - | - | 1 |
| | VI | - | - | 1 | - | - | 1 |
| *C. tropicalis* | V | - - | - | 3 | 3 | - | - |
| | VI | - | - | 3 | - | - | 3 |
| *S. cerevisiae* | V | - | - | 1 | - | - | 1 |
| | VI | - | - | 1 | - | - | 1 |
| *Trichosporon* | V | - | - | 1 | 1 | - | - |
| | VI | - | - | 1 | 1 | - | - |

| | | | | | | | |
|---|---|---|---|---|---|---|---|
| * : nombre de souches, "-" = 0 | | | | | | | |

Comme cela ressort du tableau III ci-dessus, l'apport du composé de type amide permet une détection spécifique des souches de *C. albicans.* En effet, seules les souches de *C. albicans,* ainsi qu'une souche de *Trichosporon* après 48 heures d'incubation uniquement, produisent des colonies colorées sur le milieu selon l'invention. Les souches de *C. tropicalis* qui sont bleues sur le Milieu V donnent des colonies incolores sur le Milieu VI. L'ensemble de ces deux milieux permet donc également une identification spécifique des levures de l'espèce *C. tropicalis* puisqu'après 48 heures d'incubation, elles sont les seules à être positives sur le milieu V et négatives sur le milieu VI.

### Exemple 4 :

Des essais ont été réalisés pour tester l'effet d'un mélange de composés amidés sur l'activité hexosaminidase des levures en présence d'un activateur spécifique de cette enzyme.

L'expérience de l'exemple 3 a été reproduite mais en ajoutant au milieu VI de la formamide à 0,5 g/l de milieu final (milieu VIII), le milieu VII étant identique au milieu V de l'exemple 3. Les souches étudiées sont les mêmes que dans l'exemple 3. Elles ont été directement cultivées en boîte de Pétri. Les boîtes ont été incubées à 37°C pendant 48 heures. Les colonies formées ont été examinées visuellement, respectivement après 24 et 48 heures d'incubation selon les interprétations de l'exemple 1.

Les résultats sont présentés dans le tableau IV ci-après :

**TABLEAU IV**

| | | Coloration | | | | | |
|---|---|---|---|---|---|---|---|
| | | à 24 heures | | | à 48 heures | | |
| Espèces | Milieu | Forte | Faible | Nulle | Forte | Faible | Nulle |
| *C. albicans* | VII | 2* | 1 | - | 3 | - | - |
| | VIII | 2 | 1 | - | 3 | - | - |
| *C. glabrata* | VII | - | - | 2 | - | - | 2 |
| | VIII | - | - | 2 | - | - | 2 |
| *C. krusei* | VII | - | - | 1 | - | - | 1 |
| | VIII | - | - | 1 | - | - | 1 |
| *C. parapsilosis* | VII | - | - | 1 | - | - | 1 |
| | VIII | - | - | 1 | - | - | 1 |
| *C. tropicalis* | VII | - | - | 3 | 3 | - | - |
| | VIII | - | - | 3 | - | - | 3 |
| *S. cerevisiae* | VII | - | - | 1 | - | - | 1 |
| | VIII | - | - | 1 | - | - | 1 |
| *Trichosporon* | VII | - | - | 1 | 1 | - | - |
| | VIII | - | - | 1 | - | 1 | - |

| | | | | | | | |
|---|---|---|---|---|---|---|---|
| * : nombre de souches, "-" = 0 | | | | | | | |

Comme cela ressort du tableau IV ci-dessus, l'apport d'un second composé de type amide permet une détection encore plus spécifique des souches de *C. albicans.* En effet, seules les souches de *C. albicans* produisent des colonies significativement colorées sur le milieu selon l'invention. Les souches de *C. tropicalis* qui sont bleues sur le Milieu VII donnent des colonies incolores sur le Milieu VIII et la souche de *Trichosporon* fortement colorée après 48 heures d'incubation sur le milieu VII ne l'est plus que très faiblement sur le milieu VIII.

### Exemple 5 :

Des essais ont été réalisés pour tester l'intérêt de combiner un substrat d'hexosaminidase et un substrat de β-glucosidase dans des milieux pour l'isolement et l'identification des levures.

Au milieu I de l'exemple 1, il a été ajouté un substrat de β-glucosidase, le 6-Chloro-3-indolyl-β-D-glucoside, à 0,07 g/l (milieu IX). A ce milieu, il a été ajouté soit un activateur d'hexosaminidase (N-Acétyl-glucosamine) à 1g/l (milieu X), soit un inhibiteur de l'hexosaminidase des *C. tropicalis* (Acétamide) à 1 g/l (milieu XI), soit une combinaison de l'activateur et l'inhibiteur précités aux mêmes concentrations (milieu XII).

Sur ces quatre milieux, dix-huit souches de levures ont été directement cultivées en boîtes de Pétri. Les souches, provenant de la collection de la demanderesse, appartiennent aux espèces suivantes : *C. albicans* (3 souches), *C. glabrata* (2 souches), *C. guilliermondii* (2 souches), *C. kefyr* (2 souches), *C. krusei* (1 souche), *C. lusitaniae* (2 souches), *C. parapsilosis* (1 souche), *C. tropicalis* (3 souches), *Saccharomyces cerevisiae* (1 souche), *Trichosporon spp.* (1 souche). Les boîtes ont été incubées à 37°C pendant 48 heures. Les colonies formées ont été examinées visuellement, respectivement après 24 et 48 heures d'incubation selon les interprétations suivantes :
- les colonies bleues correspondent à des souches produisant la N-acétyl-β-D-glucosaminidase appartenant *a priori* à l'espèce *C. albicans ;*
- les colonies roses correspondent à des souches produisant la β―D―glucosidase appartenant *a priori* aux espèces *C. guilliermondii, C. kefyr, C. lusitaniae,* et *C. tropicalis ;*
- les colonies mauves correspondent à des souches produisant les deux activités enzymatiques ;
- les colonies blanches correspondent aux souches ne produisant aucune des enzymes précitées ou dont ces enzymes sont inhibées, elles appartiennent donc à d'autres espèces de levures qui seront alors à identifier à l'aide des techniques habituelles.

Les résultats sont présentés dans le tableau V ci-après :

**TABLEAU V**

| | | Coloration | | | | | |
|---|---|---|---|---|---|---|---|
| | | à 24 heures | | | à 48 heures | | |
| Espèces | Milieu | Forte | Faible | Nulle | Forte | Faible | Nulle |
| *C. albicans* | IX | 1-bleue | 2-bleue | - | 3-bleue | - | - |
| | X | 2-bleue | 1-bleue | - | 3-bleue | - | - |
| | XI | 1-bleue | 2-bleue | - | 3-bleue | - | - |
| | XII | 2-bleue | 1-bleue | - | 3-bleue | - | - |
| *C. glabrata* | IX | - | - | 2 | - | - | 2 |
| | X | - | - | 2 | - | - | 2 |
| | XI | - | - | 2 | - | - | 2 |
| | XII | - | - | 2 | - | - | 2 |
| *C. guilliermondii* | IX | - | - | 2 | 2-rose | - | |
| | X | - | - | 2 | 2-rose | - | 2 |
| | XI | - | - | 2 | 2-rose | - | 2 |
| | XII | - | - | 2 | - | 2-rose | 2 |
| *C. kefyr* | IX | - | 2-rose | 2 | 2-rose | - | 2 |
| | X | - | 2-rose | 2 | 2-rose | - | 2 |
| | XI | - | 2-rose | 2 | 2-rose | - | 2 |
| | XII | - | 2-rose | 2 | 2-rose | - | 2 |
| *C. krusei* | IX | - | - | 1 | - | - | 1 |
| | X | - | - | 1 | - | - | 1 |
| | XI | - | - | 1 | - | - | 1 |
| | XII | - | - | 1 | - | - | 1 |
| *C. lusitaniae* | IX | - | - | 2 | 1-rose | 1-rose | 2 |
| | X | - | - | 2 | 2-rose | - | 2 |
| | XI | - | - | 2 | 1-rose | 1-rose | 2 |
| | XII | - | - | 2 | 2-rose | - | 2 |
| *C. parapsilosis* | IX | - | - | 1 | - | - | 1 |
| | X | - | - | 1 | - | - | 1 |
| | XI | - | - | 1 | - | - | 1 |
| | XII | - | - | 1 | - | - | 1 |
| *C. tropicalis* | IX | 2-rose | 1-rose | | 2-mauve | 1-rose | - |
| | X | 2-rose | 1-rose | | 2-mauve | 1-rose | 3 |
| | XI | 2-rose | 1-rose | | 3-rose | - | 3 |
| | XII | 2-rose | 1-rose | | 3-rose | - | 3 |
| *S . cerev-isiae* | IX | - | - | 1 | - | - | 1 |
| | X | - | - | 1 | - | - | 1 |
| | XI | - | - | 1 | - | - | 1 |
| | XII | - | - | 1 | - | - | 1 |
| *Trichosporon* | IX | - | - | 1 | 1 | - | - |
| | X | - | - | 1 | - | - | 1 |
| | XI | - | - | 1 | - | - | 1 |
| | XII | - | - | 1 | - | 1 | - |

| | | | | | | | |
|---|---|---|---|---|---|---|---|
| * : nombre de souches-couleur des colonies, "-" = 0 | | | | | | | |

Comme cela ressort du tableau V ci-dessus, l'apport d'une combinaison d'un substrat d'hexosaminidase et d'un substrat de β-glucosidase permet une détection d'un nombre plus important d'espèces de levure. En effet, il est possible sur les milieux selon l'invention de distinguer les souches de *C. albicans* d'une part, celles de *C. guilliermondii, C. kefyr, C. lusitaniae,* et *C. tropicalis* de l'autre, des autres espèces de levures. Les milieux X, XI et XII, illustre l'intérêt d'associer cette combinaison de substrat à un activateur d'hexosaminidase, à un inhibiteur spécifique de l'hexosaminidase des souches de *C. tropicalis* ou au mélange des deux. Sur le milieu X la détection des souches de *C. albicans* est plus rapide que sur le milieu IX, sur le milieu XI, la différence entre les souches de *C. albicans* et celles de *C. tropicalis* est plus nette et le milieu XII combine les avantages des milieux X et XI.

## Revendications

1. Milieu de culture pour l'identification spécifique et/ou la différenciation des levures *Candida albicans* et *Candida tropicalis* comprenant un substrat chromogène ou fluorigène, susceptible d'être hydrolysé par une enzyme du groupe des hexosaminidases, **caractérisé en ce que** le milieu comprend en outre au moins un composé sélectivement inhibiteur de l'activité hexosaminidase de *C. tropicalis.*

2. Milieu, selon la revendication 1, **caractérisé en ce que** le composé sélectivement inhibiteur est une amide de formule (I) :
(I) R-(CO-NR'R'')ₙ
dans laquelle, premièrement, soit R, R' et R" sont, indépendamment les uns des autres, constitués par :
- un atome d'hydrogène,
- une chaîne hydrocarbonée, saturée ou insaturée, aliphatique ou cyclique, comportant éventuellement au moins un hétéroatome,
soit chacun des R et/ou R' et/ou R" forment ensemble une chaîne hydrocarbonée cyclique, saturée ou insaturée, comportant éventuellement au moins un hétéroatome,
deuxièmement, n est un nombre entier supérieur ou égal à 1.

3. Milieu, selon la revendication 1, **caractérisé en ce que** le composé sélectivement inhibiteur est une amide de formule (I) :
(I) R-(CO-NR'R'')ₙ
dans laquelle, premièrement, soit R, R' et R" sont, indépendamment les uns des autres, constitués par :
- un atome d'hydrogène,
- une chaîne hydrocarbonée, saturée ou insaturée, aliphatique ou cyclique, éventuellement interrompue par au moins un hétéroatome,
soit chacun des R et/ou R' et/ou R" forment ensemble une chaîne hydrocarbonée cyclique, saturée ou insaturée, comportant éventuellement au moins un hétéroatome,
deuxièmement, n est un nombre entier supérieur ou égal à 1.

4. Milieu, selon les revendications 1 à 3, **caractérisé en ce que** le composé sélectivement inhibiteur est une amide de formule (I) :
(I) R-(CO-NR'R'')ₙ
dans laquelle, premièrement, R, R' et R" sont, indépendamment les uns des autres, constitués par :
- un atome d'hydrogène,
- une chaîne hydrocarbonée aliphatique,
et, deuxièmement, n est égal à 1 ou 2.

5. Milieu, selon les revendications 1 à 4, **caractérisé en ce que** le composé sélectivement inhibiteur est une acétamide.

6. Milieu, selon les revendications 1 à 5, **caractérisé en ce qu'**il comprend un activateur spécifique de l'enzyme hexosaminidase de *C*. *albicans.*

7. Milieu, selon la revendication 6, **caractérisé en ce que** l'activateur spécifique de l'enzyme hexosaminidase est la N-acétyl-glucosamine.

8. Milieu, selon les revendications précédentes, **caractérisé en ce qu'**il comprend un mélange de composés sélectivement inhibiteurs.

9. Milieu, selon la revendication 8, **caractérisé en ce que** le mélange de composés sélectivement inhibiteurs est constitué d'acétamide et de formamide.

10. Milieu, selon les revendications 1 et 9, **caractérisé en ce que** le milieu est gélifié et comprend pour 1 litre :
- peptones ou mélange de peptones 0,01-40 g
- extrait de levure 0,01-40 g
- glucose (source de carbone) 0-10 g
- tampon phosphate (pH entre 5 et 8,5), 2,5-100 mM
- 5-Bromo-4-chloro-3-indolyl-N-acétyl--β-D-glucosaminide (Biosynth) 20-600.10⁻⁶M
- acétamide (Sigma) 0,01-20g
- inhibiteur de bactéries 0-20g
- agar 11-20 g

11. Milieu selon les revendications 9 et 10 comprenant de plus de la N-acétyl-glucosamine à 1,0 g/l.

12. Milieu selon les revendications 10 et 11 comprenant de plus de la formamide à 0,5 g/l.

13. Milieu pour l'identification spécifique et/ou la différenciation des levures *Candida albicans* et *Candida tropicalis* selon l'une quelconque des revendications 1 à 12, **caractérisé par le fait qu'**il comprend un substrat, chromogène ou fluorigène, susceptible d'être hydrolysé par une enzyme du groupe des glucosidases.

14. Milieu, selon la revendication 13, dans lequel chaque substrat est constitué d'une partie spécifique de l'enzyme et d'une partie marqueur, **caractérisé par le fait que** la partie marqueur du premier substrat est différente de la partie marqueur du second substrat.

15. Milieu, selon l'une quelconque des revendications 13 ou 14, **caractérisé par le fait qu'**il comprend un activateur d'hexosaminidase.

16. Milieu, selon la revendication 15, **caractérisé par le fait que** l'activateur est constitué par une hexosamine et/ou une hexosaminidine.

17. Milieu, selon l'une quelconque des revendications 13 à 16, **caractérisé par le fait que** le substrat d'hexosaminidinase est constitué par un dérivé d'indoxyl et/ou que le substrat de glucosidase est constitué par un dérivé d'indoxyl.

18. Milieu, selon l'une quelconque des revendications 1 à 17, **caractérisé par le fait que** le milieu est liquide ou gélifié.

19. Procédé d'analyse microbiologique pour identifier sélectivement la levure *C. albicans* et/ou *C. tropicalis* et/ou différencier les levures *C. albicans* et *C. tropicalis,* **caractérisé en ce que** l'on met directement l'échantillon à analyser au contact d'au moins un milieu d'identification selon l'une quelconque des revendications 1 à 12.

20. Procédé d'analyse microbiologique pour détecter et identifier sélectivement certaines espèces de levures *Candida,* **caractérisé en ce que** l'on met directement l'échantillon en contact avec un milieu selon l'une quelconque des revendications 13 ou 18, que l'on attend que des colorations apparaissent dans le milieu et que l'on identifie, par des différences de colorations, les *C. albicans* par rapport, d'une part, aux *C. guilliermondii, C. kefyr, C. lusitaniae* et/ou *C. tropicalis* et, d'autre part, aux autres *Candida,* ainsi que les *C. guilliermondii, C. kefyr, C. lusitaniae* et/ou *C. tropicalis* par rapport aux autres *Candida.*

21. Procédé, selon la revendication 20, **caractérisé en ce que** l'on identifie *C. albicans, C. guilliermondii, C. kefyr, C. lusitaniae* et/ou *C. tropicalis* par rapport aux autres *Candida,* lorsque le milieu contient :
- un substrat d'hexosaminidase, et/ou
- un substrat de glucosidase, et/ou
- un activateur d'hexosaminidase, et/ou
- inhibiteur d'hexosaminidase.

22. Procédé, selon l'une quelconque des revendications 20 à 21, **caractérisé en ce que** l'on identifie *C. albicans* par rapport aux *C. guilliermondii, C. kefyr, C. lusitaniae, C. tropicalis* et/ou aux autres *Candida,* lorsque le milieu contient
- un substrat d'hexosaminidase et un substrat de glucosidase, et/ou
- un activateur d'hexosaminidase, et/ou
- inhibiteur d'hexosaminidase.

## Claims

1. Culture medium for the specific identification and/or differentiation of *Candida albicans* and *Candida tropicalis* yeasts, comprising a chromogenic or fluorigenic substrate which can be hydrolysed by an enzyme of the hexosaminidase family, **characterized in that** the medium also comprises at least one compound which selectively inhibits the hexosaminidase activity of *C. tropicalis,*

2. Medium according to Claim 1, **characterized in that** the selective inhibitor compound is an amide of formula (I):
(I) R-(CO-NR'R")ₙ
in which, firstly, either R, R' and R", independently of each other, consist of:
- a hydrogen atom,
- a saturated or unsaturated, aliphatic or cyclic hydrocarbon-based chain optionally comprising at least one hetero atom,
or each of the radicals R and/or R' and/or R" together form a cyclic, saturated or unsaturated hydrocarbon-based chain optionally comprising at least one hetero atom,
and, secondly, n is an integer greater than or equal to 1.

3. Medium according to Claim 1, **characterized in that** the selective inhibitor compound is an amide of formula (I):
(I) R-(CO-NR'R")ₙ
in which, firstly, either R, R' and R", independently of each other, consist of:
- a hydrogen atom,
- a saturated or unsaturated, aliphatic or cyclic hydrocarbon-based chain optionally interrupted by at least one hetero atom,
or each of the radicals R and/or R' and/or R" together form a cyclic, saturated or unsaturated hydrocarbon-based chain optionally comprising at least one hetero atom,
and, secondly, n is an integer greater than or equal to 1.

4. Medium according to Claims 1 to 3, **characterized in that** the selective inhibitor compound is an amide of formula (I):
(I) R-(CO-NR'R'')ₙ
in which, firstly, R, R' and R", independently of each other, consist of:
- a hydrogen atom,
- an aliphatic hydrocarbon-based chain,
and, secondly, n is equal to 1 or 2.

5. Medium according to Claims 1 to 4, **characterized in that** the selective inhibitor compound is an acetamide.

6. Medium according to Claims 1 to 5, **characterized in that** it comprises an activator which is specific for the hexosaminidase enzyme of *C*. *albicans.*

7. Medium according to Claim 6, **characterized in that** the activator which is specific for the hcxosaminidase enzyme is N-acetylglucosamine.

8. Medium according to the preceding claims, **characterized in that** it comprises a mixture of selective inhibitor compounds.

9. Medium according to Claim 8, **characterized in that** the mixture of selective inhibitor compounds consists of acetamide and formamide.

10. Medium according to Claims 1 and 9, **characterized in that** the medium is gelled and comprises, per litre:
- peptones or a mixture of peptones 0-01-40 g
- yeast extract 0.01-40 g
- glucose (source of carbon) 0-10 g
- phosphate buffer (pH between 5 and 8.5) 2.5-100 mM
- 5-bromo-4-chloro-3-indolyl-N-acetyl-β-D-glucosaminide (Biosynth) 20-600 × 10⁻⁶ M
- acetamide 0.01-20 g
- bacterial inhibitor 0-20 g
- agar 11-20 g

11. Medium according to Claims 9 and 10, furthermore comprising N-acetylglucosamine at a concentration of 1.0 g/l.

12. Medium according to Claims 10 and 11, furthermore comprising formamide at a concentration of 0.5 g/l.

13. Medium for the specific identification and/or differentiation of *Candida albicans* and *Candida tropicalis* yeasts according to any one of Claims 1 to 12, **characterized in that** it comprises a chromogenic or fluorigenic substrate which can be hydrolysed by an enzyme from the glucosidase family.

14. Medium according to Claim 13, in which each substrate consists of a specific portion of the enzyme and of a marker portion, **characterized in that** the marker portion of the first substrate is different from the marker portion of the second substrate.

15. Medium according to either of Claims 13 and 14, **characterized in that** it comprises a hexosaminidase activator.

16. Medium according to Claim 15, **characterized in that** the activator consists of a hexosamine and/or a hexosaminidine.

17. Medium according to any one of Claims 13 to 16, **characterized in that** the hexosaminidinase substrate consists of an indoxyl derivative and/or in that the glucosidase substrate consists of an indoxyl derivative.

18. Medium according to any one of Claims 1 to 17, **characterized in that** the medium is liquid or gelled.

19. Microbiological analysis process for selectively identifying the *C. albicans* and/or *C. tropicalis* yeast and/or for differentiating *C*. *albicans* and *C. tropicalis* yeasts, **characterized in that** the sample to be analysed is placed directly in contact with at least one identification medium according to any one of CLaims 1 to 12.

20. Microbiological analysis process for detecting and selectively identifying certain species of *Candida* yeasts, which is **characterized in that** the sample is placed in direct contact with a medium according to either of Claims 13 and 18, time is allowed for colorations to appear in the medium, and identification is made, on the basis of the differences in coloration, of the *C. albicans* species from, on the one hand, the *C. guilliermondii, C. kefyr, C. lusitaniae* and/or *C. tropicalis* species, and, on the other hand, from the other *Candida* species, and of the *C. guilliermondii, C. kefyr, C. lusitaniae* and/or *C. tropicalis* species from the other *Candida* species.

21. Process according to Claim 20, **characterized in that** *C. albicans, C. guilliermondii, C. kefyr, C. lusitaniae* and/or *C. tropicalis* are identified from other *Candida* species, when the medium contains:
- a hexosaminidase substrate, and/or
- a glucosidase substrate, and/or
- a hexosaminidase activator, and/or
- a hexosaminidase inhibitor.

22. Process according to either of Claims 20 and 21, **characterized in that** *C. albicans* is identified from *C. guilliermondii, C. kefyr, C. lusitaniae, C. tropicalis* and/or other *Candida* species, when the medium contains:
- a hexosaminidase substrate and a glucosidase substrate, and/or
- a hexosaminidase activator, and/or
- a hexosaminidase inhibitor.

## Patentansprüche

1. Kulturmedium zur spezifischen Identifikation und/oder Unterscheidung der Hefen *Candida albicans* und *Candida tropicalis,* umfassend ein chromogenes oder fluorigenes Substrat, das durch ein Enzym aus der Gruppe der Hexosaminidasen hydrolysiert werden kann, **dadurch gekennzeichnet, dass** das Medium außerdem mindestens eine Verbindung enthält, die ein selektiver Inhibitor der Hexosaminidase-Aktivität von *C*. *tropicalis* ist.

2. Medium nach Anspruch 1, **dadurch gekennzeichnet, dass** die selektive Inhibitorverbindung ein Amid der Formel (I):
(I) R-(CO-NR'R'')ₙ
ist, worin erstens entweder R, R' und R" unabhängig voneinander bestehen aus:
- einem Wasserstoffatom,
- einer gesättigten oder ungesättigten, aliphatischen oder cyclischen Kohlenwasserstoffkette, die gegebenenfalls mindestens ein Heteroatom Lrägt,
oder jeder von R und/oder R' und/oder R" zusammen eine cyclische, gesättigte oder ungesättigte Kohlenwasserstoffkette bilden, die gegebenenfalls mindestens ein Heteroatom trägt,
zweitens n eine ganze Zahl größer oder gleich 1 ist.

3. Medium nach Anspruch 1, **dadurch gekennzeichnet, dass** die selektive Inhibitorverbindung ein Amid der Formel (I):
(I) R-(CO-NR'R'')ₙ
ist, worin erstens entweder R, R' und R" unabhängig voneinander bestehen aus:
- einem Wasserstoffatom,
- einer gesättigten oder ungesättigten, aliphatischen oder cyclischen Kohlenwasserstoffkette, die gegebenenfalls durch mindestens ein Heteroatom unterbrochen ist,
oder jeder von R und/oder R' und/oder R" zusammen eine cyclische, gesättigte oder ungesättigte Kohlenwasserstoffkette bilden, die gegebenenfalls mindestens ein Heteroatom trägt,
zweitens n eine ganze Zahl größer oder gleich 1 ist.

4. Medium nach den Ansprüchen 1 bis 3, **dadurch gekennzeichnet, dass** die selektive Inhibitorverbindung ein Amid der Formel (I):
(I) R-(CO-NR'R'')ₙ
ist, worin erstens entweder R, R' und R" unabhängig voneinander bestehen aus:
- einem Wasserstoffatom,
- einer aliphatischen Kohlenwasserstoffkette
und zweitens n gleich 1 oder 2 ist.

5. Medium nach den Ansprüchen 1 bis 4, **dadurch gekennzeichnet, dass** die selektive Inhibitorverbindung ein Acetamid ist.

6. Medium nach den Ansprüchen 1 bis 5, **dadurch gekennzeichnet, dass** es einen spezifischen Aktivator des Hexosaminidase-Enzyms von *C. albicans* umfasst.

7. Medium nach Anspruch 6, **dadurch gekennzeichnet, dass** der spezifische Aktivator des Hexosaminidase-Enzyms N-Acetylglucosamin ist.

8. Medium nach den vorhergehenden Ansprüchen, **dadurch gekennzeichnet, dass** es ein Gemisch von selektiven Inhibitorverbindungen enthält.

9. Medium nach Anspruch 8, **dadurch gekennzeichnet, dass** das Gemisch von selektiven Inhibitorverbindungen aus Acetamid und Formamid besteht.

10. Medium nach den Ansprüchen 1 und 9, **dadurch gekennzeichnet, dass** das Medium geliert ist und für 1 Liter Folgendes enthält:
- Peptone oder Gemisch von Peptonen 0,01-40 g
- Hefeextrakt 0,01-40 g
- Clucose (Kohlenstoffquelle) 0-10 g
- Phosphatpuffer (pH zwischen 5 und 8,5) 2,5-100 mM
- 5-Brom-4-chlor-3-indolyl-N-acetyl-β-D- 20-600·10⁻⁶ M glucosaminid (Biosynth)
- Acetamid (Sigma) 0,01-20 g
- Bakterieninhibitor 0-20 g
- Agar 11-20 g

11. Medium nach den Ansprüchen 9 und 10, das außerdem 1,0 g/l N-Acetylglucosamin enthält.

12. Medium nach den Ansprüchen 10 und 11, das außerdem 0,5 g/l. Formamid enthält.

13. Medium zur spezifischen Identifikation und/oder Unterscheidung der Hefen *Candida albicans* und *Candida tropicalis* nach einem der Ansprüche 1 bis 12, **dadurch gekennzeichnet, dass** es ein chromogenes oder fluorigenes Substrat umfasst, das durch ein Enzym aus der Gruppe der Glucosidasen hydrolysiert werden kann.

14. Medium nach Anspruch 13, wobei jedes Substrat aus einem für das Enzym spezifischen Anteil und einem Markeranteil besteht, **dadurch gekennzeichnet, dass** der Markeranteil des ersten Substrats vom Markeranteil des zweiten Substrats verschieden ist.

15. Medium nach einem der Ansprüche 13 oder 14, **dadurch gekennzeichnet, dass** es einen Hexosaminidase-Aktivator enthält.

16. Medium nach Anspruch 15, **dadurch gekennzeichnet, dass** der Aktivator aus einem Hexosamin und/oder einem Hexosaminidin besteht.

17. Medium nach einem der Ansprüche 13 bis 16, **dadurch gekennzeichnet, dass** das Hexosaminidinase-Substrat aus einem Indoxylderivat besteht und/oder dass das Gluoosidase-Substrat aus einem Indoxylderivat besteht.

18. Medium nach einem der Ansprüche 1 bis 17, **dadurch gekennzeichnet, dass** das Medium flüssig oder geliert ist.

19. Verfahren zur mikrobiologischen Analyse, um selektiv die Hefe *C. albicans* und/oder *C. tropicalis* zu identifizieren und/oder zwischen den Hefen *C. albicans* und/oder *C. tropicalis* zu unterscheiden, **dadurch gekennzeichnet, dass** die zu analysierende Probe direkt mit mindestens einem Identifikationsmedium nach einem der Ansprüche 1 bis 12 in Kontakt gebracht wird.

20. Verfahren zur mikrobiologischen Analyse, um bestimmte Spezies der *Candida-*Hefen selektiv nachzuweisen und zu identifizieren, **dadurch gekennzeichnet, dass** die Probe direkt mit einem Medium nach einem der Ansprüche 13 oder 18 in Kontakt gebracht wird, dass die Färbungen, die in dem Medium erscheinen, abgewartet werden, und dass aufgrund von Unterschieden in den Färbungen *C. albicans* einerseits im Vergleich zu *C*. *guilliermondii, C. kefyr, C. lusitaniae* und/oder *C*. *tropicalis* und andererseits zu anderen *Candida* sowie *C*. *guilliermondii, C. kefyr, C. lusitaniae* und/oder *C. tropicalis* im Vergleich zu anderen *Candida* identifiziert werden.

21. Verfahren nach Anspruch 20, **dadurch gekennzeichnet, dass** *C. albicans, C. guilliermondii, C. kefyr, C. lusitaniae* und/oder *C. tropicalis* im Vergleich zu anderen *Candida* identifiziert werden, wenn das Medium Folgendes enthält:
- ein Hexosaminidase-Substrat und/oder
- ein Glucosidase-Substrat und/oder
- einen Hexosaminidase-Aktivator und/oder
- einen Hexosaminidase-Inhibitor.

22. Verfahren nach einem der Ansprüche 20 bis 21, **dadurch gekennzeichnet, dass** *C. albicans* im Vergleich zu *C. guilliermondii, C. kefyr, C. lusitaniae, C. tropicalis* und/oder anderen *Candida* identifiziert wird, wenn das Medium Folgendes enthält:
- ein Hexosaminidase-Substrat und ein Glucosidase-Substrat und/oder
- einen Hexosaminidase-Aktivator und/oder
- einen Hexosaminidase-Inhibitor.
